# EUROPEAN PATENT APPLICATION

(11) **EP 0 779 275 A2**
(43) Date of publication of application: **18.06.1997**
(21) Application number: 96308899.2
(22) Date of filing: 09.12.1996
(51) Int. Cl.: C07C 327/22, C07D 317/30, C07D 307/32

(54) **Alpha, Alpha - Difluoro-beta -hydroxy thiol esters and their synthesis**

(30) Priority: 13.12.1995 US 8522; 15.01.1996 GB 9600757
(71) Applicant: ELI LILLY AND COMPANY, Indianapolis, Indiana 46285 (US)
(72) Inventor: Weigel, John Ashley, Lafayette, Indiana 47905 (US)
(74) Representative: Tapping, Kenneth George

(57) **Abstract**

A process to make α,α-difluoro-β-hydroxy thiol esters of formula (IIIA), 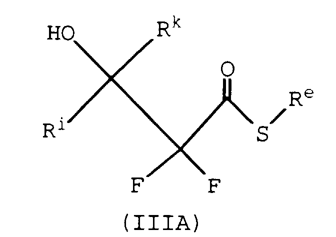 comprising reacting a difluoroethanethioate of formula (IVA) 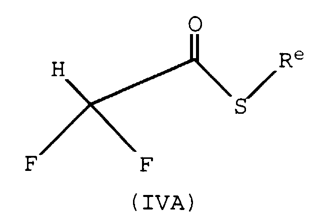 with a second reactant selected from the group consisting of aldehydes, ketones, acid halides and esters; in a solvent and in the presence of a strong base; with the proviso that the process is conducted in the absence of a catalyst and in the absence of a silyl containing compound is described and claimed.

The α,α-difluoro-β-hydroxy thiol esters can be useful in the syntheses of organic compounds including gemcitabine hydrochloride.

## Description

The invention relates to the art of organic chemistry. Specifically it relates to certain α,α-difluoro-β-hydroxy thiol esters and processes to make them.

α,α-Difluoro-β-hydroxy thiol esters are useful as intermediates in organic syntheses of valuable pharmaceutical products.

A particularly useful α,α-difluoro-β-hydroxy ester is: 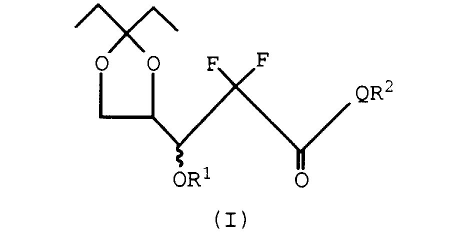 where R¹ is selected from the group consisting of H, C₁-C₄ alkyl, substituted alkyl, including silyl alkyl, trialkylsilyl, aryl, substituted aryl, including silyl aryl, R² is independently selected from alkyl and aryl groups, and Q is either S (for thiol esters) or O (for esters). Compounds of formula I are known to be useful in the synthesis of 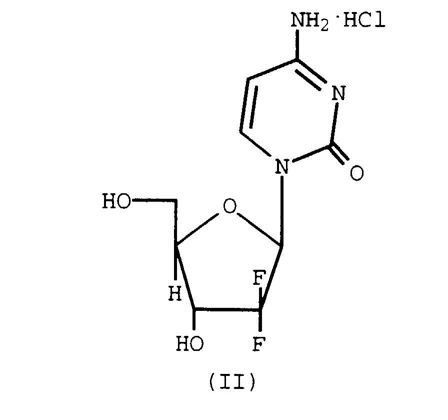 gemcitabine hydrochloride (II), a known anti-neoplastic agent.

Existing methods to make compounds of formula I where R¹ is SiR³R⁴R⁵, R² is R⁶, Q is S, and R³, R⁴, R⁵ and R⁶ are independently selected from C₁-C₄ alkyl and C₅-C₆ aryl groups are known in the art. See U.S. Patent 5,428,176 . The processes to make compounds of formula (I) as described in the '176 patent require a trialkyl(or triaryl)silylhalide and the use of a catalyst.

Existing methods to make compounds of formula I where R¹ is trialkylsilyl, R² is alkyl and Q is O, are described and claimed in European Patent Application No. 94308806.2 (Publication No. 655454). These methods also require a trialkylsilyl halide and the use of a catalyst.

It is desirable to develop alternate processes that use fewer reagents to make certain α,α-difluoro-β-hydroxy thiol esters, because the use of fewer reagents would lower the overall cost of the synthesis. It is also desirable to synthesize additional α,α-difluoro-β-hydroxy thiol ester compounds which can be useful as intermediates in organic syntheses of valuable pharmaceutical products.

A first aspect of this invention is α,α-Difluoro-β-hydroxy thiol esters of formula (III) 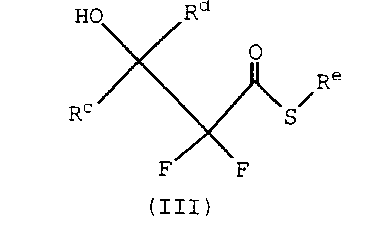 wherein:
R^{c} and R^{d} are independently selected from the group consisting of H, C₁-C₆ alkyl, substituted C₁-C₆ alkyl, *S-tert*-butyl difluorothioacet-2-yl, C₃-C₈ cycloalkyl, C₃-C₈ cycloalkenyl, substituted C₃-C₈ cycloalkyl, substituted C₃-C₈ cycloalkenyl, aryl, substituted aryl, C₆-C₁₀ fused aromatic rings, and substituted C₆-C₁₀ fused aromatic rings; or
R^{c} and R^{d} together make up a ring selected from the group consisting of C₃-C₈ cycloalkyl, C₃-C₈ cycloalkenyl, substituted C₃-C₈ cycloalkyl and substituted C₃-C₈ cycloalkenyl;
R^{e} is selected from the group consisting of C₁-C₁₀ alkyl, substituted C₁-C₁₀ alkyl, aryl, substituted aryl, C₃-C₈ cycloalkyl, C₃-C₈ cycloalkenyl, substituted C₃-C₈ cycloalkyl and substituted C₃-C₈ cycloalkenyl, C₆-C₁₀ fused aromatic rings, and substituted C₆-C₁₀ fused aromatic rings.

A second aspect of this invention is a difluoroethanethioate compound of formula (IV): 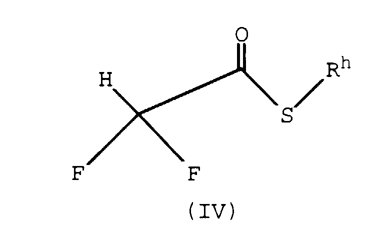 wherein R^{h} is selected from the group consisting of C₃-C₈ cycloalkenyl and substituted C₃-C₈ cycloalkenyl.

A third aspect of this invention is a process to make α,α-difluoro-β-hydroxy thiol esters of formula (IIIA), 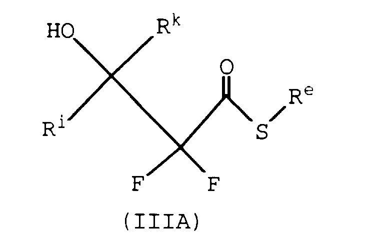 wherein:
Rⁱ and R^{k} are independently selected from the group consisting of H, C₁-C₆ alkyl, substituted C₁-C₆ alkyl, *S-tert*-butyl difluorothioacet-2-yl, C₃-C₈ cycloalkyl, C₃-C₈ cycloalkenyl, substituted C₃-C₈ cycloalkyl, substituted C₃-C₈ cycloalkenyl, aryl, substituted aryl, 1,3-dioxolan-4-yl, substituted 1,3-dioxolan-4-yl, C₆-C₁₀ fused aromatic rings, substituted C₆-C₁₀ fused aromatic rings; or
Rⁱ and R^{k} together make up a ring selected from the group consisting of C₃-C₈ cycloalkyl, C₃-C₈ cycloalkenyl, substituted C₃-C₈ cycloalkyl, and substituted C₃-C₈ cycloalkenyl;
R^{e} is selected from the group consisting of C₁-C₁₀ alkyl, substituted C₁-C₁₀ alkyl, aryl, substituted aryl, C₃-C₈ cycloalkyl, C₃-C₈ cycloalkenyl, substituted C₃-C₈ cycloalkyl, and substituted C₃-C₈ cycloalkenyl, C₆-C₁₀ fused aromatic rings, and substituted C₆-C₁₀ fused aromatic rings; comprising reacting a difluoroethanethioate of formula (IVA) 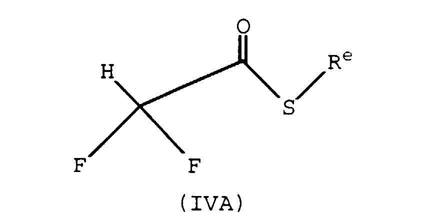 wherein
R^{e} is as defined previously;

with a second reactant selected from the group consisting of aldehydes, ketones, acid halides and esters; in a solvent and in the presence of a strong base; with the proviso that the process is conducted in the absence of a catalyst and in the absence of a silyl containing compound.

A fourth aspect of this invention is a process to make gemcitabine hydrochloride, the improvement characterized in that the lactone intermediate, 2-deoxy-2,2-difluoro-D-erythro-pentofuranose-1-ulose-3,5-dibenzoate: 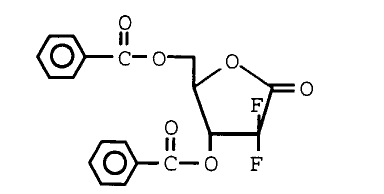 is made from D-erythro-2-Deoxy-2,2-difluoro-4,5-O-(1-ethylpropylidene) pentoic acid, tert-Butyl thioester, with said D-erythro-2-Deoxy-2,2-difluoro-4,5-O-(1-ethylpropylidene) pentoic acid, tert-Butyl thioester being made by the process of the third aspect of this invention.

The following definitions are set forth to illustrate and define the meaning and scope of the various terms used to describe the invention herein.

The term "alkyl" by itself or as part of another substituent means a monovalent compound having the stated number of carbon atoms containing only carbon and hydrogen, and which my be straight or branched chain. The term is exemplified by compounds containing from 1 to 10 carbon atoms, such as, but not limited to, methyl, ethyl, propyl, isopropyl, n-butyl, isobutyl, sec-butyl and tert-butyl (t-butyl), n-pentyl, iso-pentyl, n-hexyl, isohexyl, heptyl, octyl, nonyl and decyl; "C₁-C₄ alkyl" refers to alkyl compound of from 1-4 carbon atoms. "C₁-C₆ alkyl" refers to alkyl compounds of from 1-6 carbon atoms. "C₁-C₁₀" alkyl refers to alkyl compounds of from 1-10 carbon atoms.

The term "substituted" means one to three hydrogens on the structure have been replaced with a like number of moieties independently selected from the group consisting of bromo, chloro, iodo, fluoro, C₁-C₁₀ alkyl,alkoxy, **-**COOH**,** amino or hydroxyl, with the proviso that any substituted structure must be so configured that it is sterically feasible, affords a stable structure and is capable of reacting as described herein. ·

The term "C₁-C₆ alkoxy" refers to monovalent structures of the formula -O-(C₁-C₆ alkyl). C₁-C₆ alkoxy includes, but is not limited to, methoxy (-OCH₃), ethoxy (-OCH₂CH₃),
propoxy(-OCH₂CH₂CH₃), butoxy(-OCH₂CH₂CH₂CH₃),
pentoxy(-OCH₂CH₂CH₂CH₂CH₃) and
hexoxy(-OCH₂CH₂CH₂CH₂CH₂CH₃).

To be "aromatic" a ring must contain one or more groups of atoms in a cyclic array that contains clouds of delocalized π electrons above and below the plane of the atoms; furthermore, the π clouds must contain a total of (4q+2) π electrons, where q is any positive integer.

The term "aryl" refers to:
any 5 or 6 membered aromatic ring that will afford a stable structure containing all carbon atoms; or containing carbon atoms and: one or two nitrogen atoms; one sulfur atom; one oxygen atom; one nitrogen and one sulfur atom; one oxygen atom and one sulfur atom; or one nitrogen and one oxygen atom. The 5-membered ring has one or two double bonds and the 6-membered ring has two or three double bonds. Examples of aryl structures are:

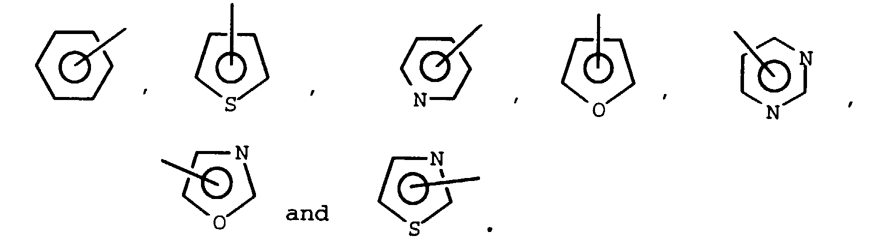

The term "C₆-C₁₀ fused aromatic rings" refers to two fused aromatic rings that have at least six and at most ten carbon atoms in the rings. Fused aromatic rings are monovalent, with the bond to the rest of the structure being attached to any available ring carbon. Fused aromatic rings are carbocyclic or contain from one to four heteroatoms independently selected from the group consisting of nitrogen, oxygen and sulfur. Fused aromatic rings are configured as follows: when there are six carbon atoms present in the rings, there are at least four heteroatoms in the rings and all of these heteroatoms are nitrogen; when there are seven carbon atoms present in the rings, there are at least three heteroatoms present in the rings, only one of which may be sulfur; when there are eight carbon atoms present in the rings, there are at least two heteroatoms, which may be the same or different, present in the rings; when there are nine carbon atoms in the rings there may or may not be one heteroatom in one of the rings; and when there are ten carbon atoms in the rings there are no heteroatoms in the rings. Examples of some "C₆-C₁₀ fused aromatic rings" include, but are not limited to, the following structures:

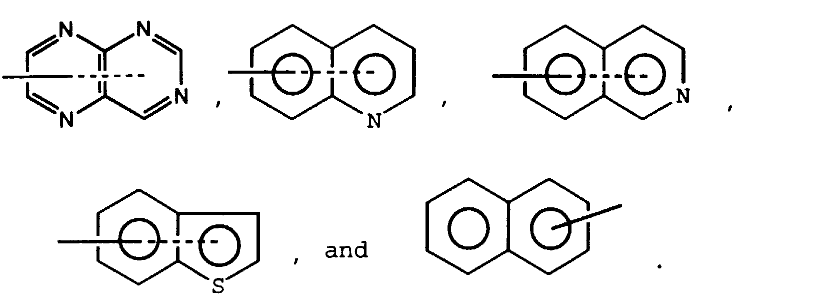

The solid bond that becomes a dotted line bond, present in the above structures indicates that the bond can be attached to any available carbon in any ring that the solid-dotted line intersects.

The term "C₃-C₈ cycloalkyl" refers to saturated carbocyclic ring structures containing from 3 to 8 carbon atoms. Examples of some "C₃-C₈ cycloalkyl" rings include, cyclopropyl, cyclobutyl, cyclopentyl, cyclohexyl, cycloheptyl and cyclooctyl.

The term "C₃-C₈ cycloalkenyl" refers to carbocyclic ring structures containing from 3 to 8 carbon atoms and one double bond. Examples of some "C₃-C₈ cycloalkenyl" rings include, cyclopropenyl, cyclobutenyl, cyclopentenyl, cyclohexenyl, cycloheptenyl and cyclooctenyl.

The term "halo" refers to bromo, iodo, fluoro and chloro.

Preferred α,α-Difluoro-β-hydroxy thiol esters compounds of Formula (III) are selected from the group consisting of:
*S-tert* Butyl 2,2-Difluoro-3-hydroxy-3-phenylpropanethioate;
*S- tert* Butyl 2,2-Difluoro-3-hydroxy-3-(4-methoxyphenyl)-propanethioate;
*S-tert* Butyl 2,2-Difluoro-3-hydroxy-octanethioate;
*S-tert* Butyl 2,2-Difluoro-3-hydroxy-4,4-dimethylpentanethioate;
*S-tert* Butyl 2,2-Difluoro-3-hydroxy-4-methylpentanethioate;
*S-tert* Butyl 2,2-Difluoro-3-hydroxy-3-phenylbutanethioate;
*Bis(S-tert* Butyl) 2,2,4,4-Tetrafluoro-3-hydroxy-3-phenylpentane-1,5-dithioate;
*S-tert* Butyl 1-(2-(2,2-difluoroethane-thioate))cyclohex-2-en-1-ol;
D-*erythro*-2-Deoxy-2,2-difluoro-4,5-O-(1-ethylpropylidene)pentonic acid, *tert*-Butyl Thiolester; and
D-*threo*-2-Deoxy-2,2-difluoro-4,5-O-(1-ethylpropylidene)pentonic acid, *tert*-Butyl Thiolester.

Preferred difluoroethanethioate compounds of formula (IV) are selected from the group consisting of:
S-cyclobuten-3-yl difluoroethanethioate;
S-cyclopenten-1-yl difluoroethanethioate;
S-cyclopenten-3-yl difluoroethanethioate;
S-cyclopenten-4-yl difluoroethanethioate;
S-cyclohexen-1-yl difluoroethanethioate;
S-2-hydroxycyclohexen-3-yl difluoroethanethioate;
S-cyclohexen-4-yl difluoroethanethioate;
S-cyclohepten-1-yl difluoroethanethioate;
S-cyclohepten-3-yl difluoroethanethioate;
S-cyclohepten-4-yl difluoroethanethioate;
S-cyclohepten-5-yl difluoroethanethioate;
S-cycloocten-1-yl difluoroethanethioate;
S-cycloocten-3-yl difluoroethanethioate;
S-cycloocten-4-yl difluoroethanethioate and
S-cycloocten-5-yl difluoroethanethioate.

The first step in the process of the present invention to make the α,α-difluoro-β-hydroxy thiol esters of the present invention is the synthesis of a difluoroethanethioate of Formula (IVA). A preferred method to synthesize a difluoroethanethioate of Formula (IVA) is as follows:

Step 1. At a temperature of between about 20°C and about 100°C, under a suitable inert atmosphere such as nitrogen, argon or neon, add difluoroacetic acid or difluoroacetic anhydride dropwise to a solution of chloride containing compound in an aprotic solvent. The chloride containing compound may be any compound that will, under suitable conditions, release chloride to form an acid chloride. Suitable chloride containing compounds include oxaloyl chloride, thionyl chloride, phosgene, phosphorous oxychloride, phosphorous trichloride and phosphorous pentachloride. The preferred chloride containing compound is oxaloyl chloride. Any aprotic solvent may be used; for example: acetonitrile, tetrahydrofuran, dichloromethane, toluene, glyme and xylene are all useful. A preferred aprotic solvent is acetonitrile. The temperature selected depends upon the choice of chloride containing compound and solvent with about 25°C being suitable when oxaloyl chloride in acetonitrile is used. From the beginning of the reaction, and at any time thereafter, a catalytic amount of a suitable Lewis acid catalyst may be added. One such suitable Lewis acid catalyst is cobalt (II) chloride. The use of a catalyst in this process is optional.

Step 2. Allow the reactants of step 1 to react from between one and ten hours. The overall reaction time is dependent upon temperature with the higher temperatures having the faster reaction times. Add a sufficient amount of a suitable thiol compound in a dropwise manner, with said suitable thiol compound being of the formula R^{e}-SH, where R^{e} is as defined previously, and selected such that the desired R^{e} component is introduced into the difluoroethanethioate product. For example when R^{e} is tert-butyl the suitable thiol is 2-methyl-2-propanethiol. A "sufficient amount" is at least one molar equivalent based upon the difluoro starting material.

Step 3. Stir reaction mixture for a sufficient amount of time. A sufficient amount of time could be any time up to 24 hours. Add additional suitable thiol compound, if necessary. The temperature of the reaction mixture can be anywhere between about 20°C and about 100°C with 25°C being the preferred reaction temperature.

Step 4. Perform a standard workup using standard techniques. One such workup is as follows: Pour the reaction mixture over a suitable water immiscible solvent such as diethyl ether (Et₂O) and then wash with a suitable basic aqueous solution such as saturated sodium bicarbonate (NaHCO₃) in water.

During step 4, the reaction mixture divides into two layers with the desired product contained in the organic layer. The mostly aqueous layer containing the undesired material is separated and discarded in an environmentally sound manner. The layer containing the desired product is dried over a suitable drying reagent such as sodium sulfate (Na₂SO₄).

Once the substituted difluoroethanethioate is made it may be kept either at room temperature or, more preferred, kept in a refrigerator at a temperature below 25°C until it is needed. The substituted difluoroethanethioate (IVA) is used to begin the process to make the α,α-difluoro-β-hydroxy thiol esters (IIIA) of the instant invention. The preferred substituted difluoroethanethioate for the synthesis of gemcitabine hydrochloride is *S-tert* butyl difluoroethanethioate.

One synthetic route to α,α-difluoro-β-hydroxy thiol esters is to react a substituted difluoroethanethioate (IV) with a second reactant selected from the group consisting of aldehydes, ketones, acid halides and esters in a solvent and in the presence of a strong base; with the proviso that the process is conducted in the absence of a catalyst and in the absence of a silyl containing compound.

The aldehyde or ketone is of formula (V): 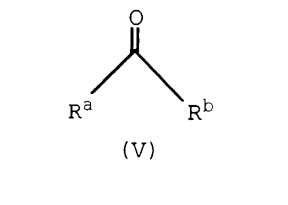 wherein R^{a} and R^{b} are independently selected from the group consisting of H, C₁-C₆ alkyl, substituted C₁-C₆ alkyl, C₃-C₈ cycloalkyl, substituted C₃-C₈ cycloalkyl, C₃-C₈ cycloalkenyl, substituted C₃-C₈ cycloalkenyl, aryl, substituted aryl, 1,3-dioxolan-4-yl, substituted 1,3-dioxolan-4-yl, C₆-C₁₀ fused aromatic rings and substituted C₆-C₁₀ fused aromatic rings, or R^{a} and R^{b} together make up a ring selected from the group consisting of C₃-C₈ cycloalkyl, substituted C₃-C₈ cycloalkyl, C₃-C₈ cycloalkenyl and substituted C₃-C₈ cycloalkenyl.

The acid halides are of formula (VI) 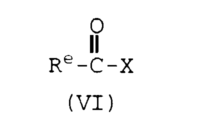 where X is Cl, Fl, Br or I and R^{e} has the same definition as before.

The esters are of formula (VII) 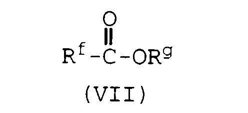 where R^{f} and R^{g} are independently R^{e}, where R^{e} has the same definition as before.

Without intending to be a limiting list, examples of suitable aldehydes, ketones, acid halides and esters are as follows.

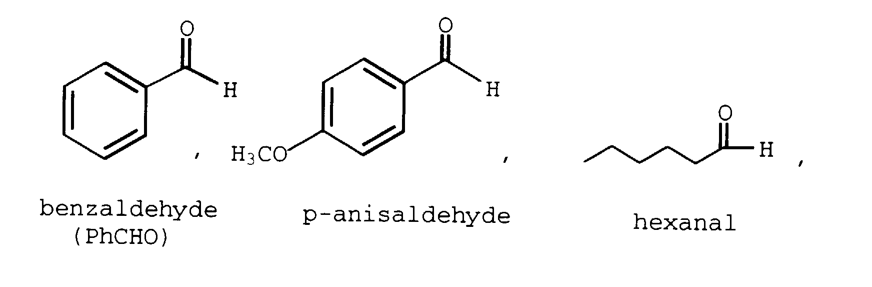

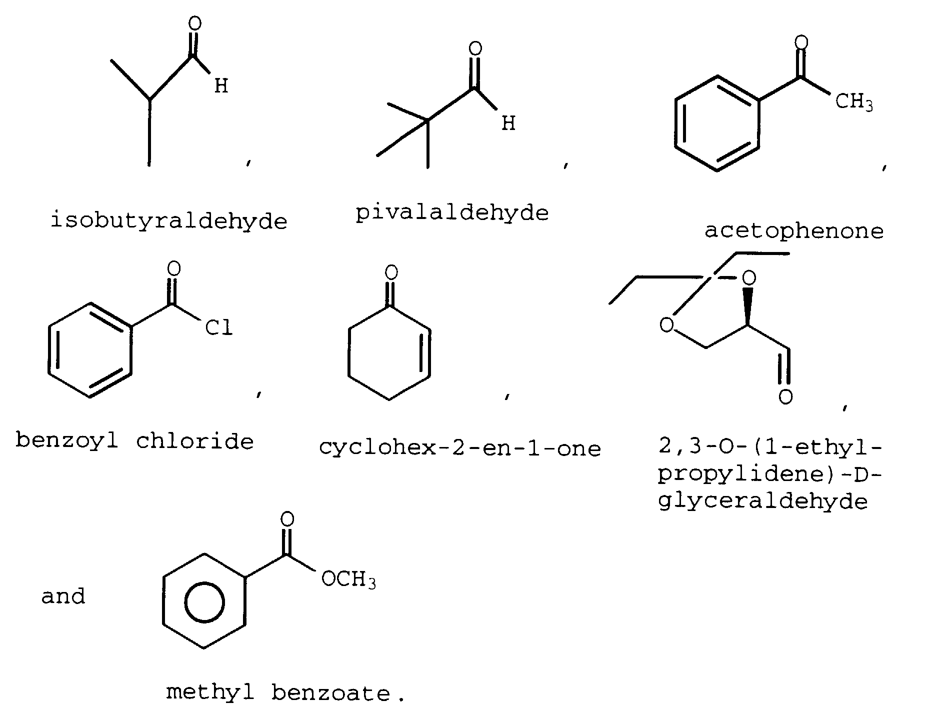

Methods to make all of these aldehydes, ketones, acid halides and esters are known in the art of organic chemistry.

The preferred second reactant, for use in the overall synthesis of gemcitabine hydrochloride, is 2,3-O-(3-pentylidene)-D-glyceraldehyde.

The synthesis involves reacting a difluoroethanethioate (IVA) with a second reactant (of formula V, VI or VII) in a solvent, in the presence of a strong base; with the proviso that the process is conducted in the absence of a catalyst and in the absence of a silyl containing compound.

The preferred order of addition or reagents is to first add the substituted difluoroethanethioate to the solvent, then add the strong base and allow the three components to react for a sufficient length of time before adding the second reactant to the reaction mixture. Of course, this synthesis is preferably conducted under a suitable inert atmosphere such as nitrogen, argon or neon.

Suitable solvents for the synthesis include hydrocarbons, nitriles and ethers. These solvents include, but are not limited to, toluene, xylene, glyme, tetrahydrofuran, acetonitrile, hexane, heptane, diethyl ether and many other solvents of the general class. The most preferred solvent for this reaction is toluene.

The strong base is any suitable strong base such as amides, alkoxides and hydrides. Suitable strong bases include lithium diisopropylamide (LDA), lithium hexamethylsilazide, triethylamine, pyridine and n-butyl lithium. The preferred solvent is LDA.

The sufficient length of time for the substituted difluoroethanethioate, solvent and strong base to react is anywhere between about thirty seconds and about 1 hour. A preferred length of time is about 2 minutes. If desirable, the reaction mixture is capable of being stabilized at this point, preferably by keeping it at a temperature below 25°C, and held indefinitely until needed.

The temperature of the substituted difluoro-ethanethioate/solvent mixture is lowered to between at least about -100°C and about 25°C prior to the addition of the strong base. The preferred temperature is about -78°C. After the strong base and the second reactant are added the reaction is allowed to precede at about the same temperature for between about five minutes and about 24 hours.

When the reaction has reached a suitable ending point the reaction mixture may be warmed to about room temperature (about 25°C). If necessary, the reaction can be quenched by addition of a suitable quenching agent, such as, phosphate buffer. Once the reaction is ended, the organic layer is separated and concentrated under reduced pressure. The crude desired product can be purified by chromatography. The undesired layer can be disposed of in an environmentally sound manner.

This procedure was followed to make the compounds of the claimed invention.

The α,α-difluoro-β-hydroxy thiol esters of the claimed invention can be useful as intermediates in organic syntheses of valuable pharmaceutical products.

After D-erythro (a.k.a. anti-) 2-Deoxy-2,2-difluoro-4,5-O-(1-ethylpropylidene) pentoic acid, tert-Butyl thioester is made it can be converted to a lactone of the formula: 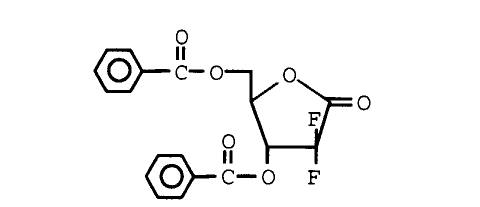 by following the procedures described in U.S. Patent No. 4,526,988 (columns 7,8) which is incorporated by reference. The lactone is then converted to an alcohol, forming a protected 2-deoxy-2,2-difluororibose compound. This protected 2-deoxy-2,2-difluororibose compound can be reacted with a nucleobase, according to the process(es) described in European Patent Application No. 93304817.5 (Publication No. 577 303 Al), European Patent Application No. 84301463.0 (Publication No. 122 707), and in European Patent Application No. 88307750.5 (Publication No. 306 190), to form nucleosides, including pharmaceutically acceptable salts of nucleosides, with said nucleosides, and pharmaceutically acceptable salts of said nucleosides, having utility as pharmaceutical products. One such nucleoside is gemcitabine hydrochloride: 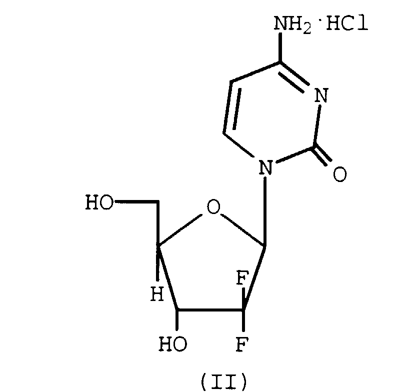 which is a commercially available (sold under the trademark, GEMZAR® by Eli Lilly and Company, Indianapolis, IN) anti-neoplastic drug.

The following examples are illustrative only and are not intended to limit the scope of the invention in any way.

### Examples

The terms and abbreviations used in the instant examples have their normal meanings unless otherwise designated. For example: "°C" refers to degrees Celsius; "N" refers to normal or normality; "mol" refers to mole or moles; "mmol" refers to millimole or millimoles; "g" refers to gram or grams; "mg" refers to milligrams; "ml" refers to milliliter or milliliters; "mp" refers to melting point; "M" refers to molar or molarity; "mm Hg" refers to millimeters of mercury; "Mass spec." refers to mass spectrometry; "IR" refers to infrared spectroscopy; and "NMR" refers to nuclear magnetic resonance spectroscopy.

Unless otherwise noted, all chemicals were reagent grade materials from commercial suppliers and were used without further purification. All reactions were conducted under a nitrogen atmosphere. Melting points are uncorrected. ¹H NMR spectra were recorded at 300 MHz and were internally referenced to residual CHCl₃ (7.24 ppm). ¹³C NMR spectra were recorded at 75.5 MHz and were referenced to CHCl₃ (77.0 ppm). ¹⁹F NMR spectra were recorded at 282.4 MHz and were referenced to internal C₆F₆ (-162.9 ppm). Assignments were derived from COSY and H/C correlation spectra and from consideration of the F/C coupling patterns observed in the carbon spectra. IR spectra were recorded as thin films.

### Example 1 S-tert Butyl Difluoroethanethioate

To a solution of oxaloyl chloride (9.08 mL, 104 mmol) in acetonitrile (ACN) (50 mL) at 25 °C was added dropwise difluoroacetic acid (6.55 mL, 104 mmol). After 3 hours, 2-methyl-2-propanethiol (11.74 mL, 104 mmol) was added dropwise followed by cobalt(II) chloride (10 mg). After being stirred at room temperature for 18 hours, additional 2-methyl-2-propanethiol (4.0 mL, 35.4 mmol) was added. After 2 hours, the solution was poured onto Et₂O (500 mL) and washed with saturated NaHCO₃ (2 x 300 mL) and H₂O (2 x 300 mL). The organic layer was dried over Na₂SO₄, and the solvent was removed under reduced pressure to give a red oil. Vacuum distillation at 30 mm Hg gave S-tert butyl difluoroethane-thioate (8.26 g, 47%) as a colorless oil: bp 59-63 °C (30 mm Hg); ¹H NMR (CDCl₃) δ 1.48 (s, 9H), 5.67 (t, 1H, J = 54 Hz); ¹³C NMR (CDCl₃) δ 29.45, 49.32, 108.94 (t, J = 255 Hz), 191.23 (t, J = 28 Hz); ¹⁹F NMR (CDCl₃) δ -123.65 (d, J = 55 Hz); IR (film, cm-¹) 2969, 1684, 1154, 1093, 1064. Anal. Calcd for C₆H₁₀F₂OS: C, 42.84; H, 5.99. Found: C, 43.06; H, 6.15.

### Example 2 General Process to Make α,α-difluoro-β-hydroxy thiol esters using S-tert butyl difluoroethanethioate as a starting material

To a solution of S-tert butyl difluoroethanethioate (VIII) (0.152 mL, 1.00 mmol) from Example 1 in toluene (10 mL) at -78 °C was added dropwise over 1 min LDA (0.550 mL of 2.0 M solution in heptane/THF/ethylbenzene, 1.10 mmol). After 2 min, the second reactant (aldehyde (V), ketone (V), acid chloride (VI) or ester (VII) (1.10 mmol)) was added dropwise. After 1 hour, the reaction was warmed to 25°C over 1 hour and quenched by addition of phosphate buffer (5 mL, 0.5 M, pH = 7). The resulting organic layer was concentrated under reduced pressure. The crude product was isolated and purified by chromatography.

### Example 3 Synthesis of S-tert Butyl 2,2-Difluoro-3-hydroxy-3-phenylpropanethioate (IX)

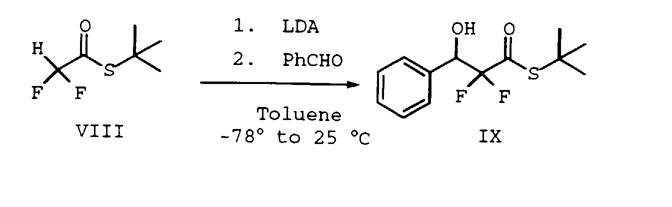

Following the procedure of Example 2, reaction of benzaldehyde (0.112 mL, 1.10 mmol) affords *S-tert* butyl 2,2-difluoro-3-hydroxy-3-phenylpropanethioate. (IX) (192 mg after isolation and purification, 70%) as a colorless oil, which was chromatographed using 10% EtOAc in hexane as eluent: ¹H NMR (CDCl₃) δ 1.46 (s, 9H), 2.52 (br, 1H), 5.16 (dd, 1H), 7.4 (m, 5H); ¹³C NMR (CDCl₃) δ 29.31, 49.05, 73.49 (t, J = 24 Hz), 114.78 (t, J = 260 Hz), 127.80, 128.32, 129.10, 134.56, 193.47 (t, J = 30 Hz); ¹⁹F NMR (CDCl₃) δ -112.88 (J_{FF} = 259 Hz, J_{FH} = 9 Hz), -119.22 (J_{FF} = 259, J_{FH} = 15 Hz); IR (film, cm⁻¹) 3442, 2967, 1673, 1660, 1456, 1367, 1194, 1078, 917.

### Example 4 S-tert Butyl 2,2-Difluoro-3-hydroxy-3-(4-methoxyphenyl)-propanethioate (X)

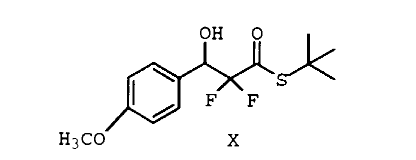

Following the procedure of Example 2, reaction of *p*-anisaldehyde (0.134 mL, 1.10 mmol) affords compound (X) (187 mg after isolation and purification, 62%) as a white solid, which was chromatographed using 5% EtOAc in hexane as eluent: mp 73-75 °C; ¹H NMR (CDCl₃) δ 1.45 (s 9H), 2.49 (br, 1H), 3.79 (s, 3H), 5.09 (dd, 1H), 6.87 (d, 2H), 7.31 (d, 2H); ¹³C NMR (CDCl₃) δ 29.34, 49.04, 55.26, 73.10 (t, J = 25 Hz), 113.78, 114.85 (t, J = 260 Hz), 126.58, 129.11, 160.19, 193.57 (t, J = 31 Hz); ¹⁹F NMR (CDCl₃) δ-113.13 (J_{FF} = 258 Hz, J_{FH} = 8 Hz), -119.35 (J_{FF} = 258, J_{FH} = 15 Hz); IR (film, cm⁻¹) 3474, 2960, 1683, 1618, 1519, 1453, 1249, 1177, 1085, 1038, 920.

### Example 5 Synthesis of S-tert Butyl 2,2-Difluoro-3-hydroxy-octanethioate (XI

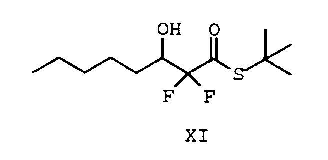

Following the procedure of Example 2, reaction of hexanal (0.132 mL, 1.10 mmol) affords compound (XI) (164 mg after isolation and purification, 61%) as a colorless oil, which was chromatographed using 10% EtOAc in hexane as eluent: ¹H NMR (CDCl₃) δ 0.88 (t, 3H), 1.30 (m, 6H), 1.51 (s, 9H), 1.60 (m, 2H), 1.99 (br, 1H), 4.01 (m, 1H); ¹³C NMR (CDCl₃) δ 13.93, 22.43, 24.88, 29.31, 29.47, 31.41, 49.10, 71.47 (t, J = 25 Hz), 115.57 (t, J = 259 Hz), 193.55 (t, J =33 Hz); ¹⁹F NMR (CDCl₃) δ -114.11 (J_{FF} = 261 Hz, J_{FH} = 9 Hz), -119.94 (J_{FF} = 261, J_{FH} = 14 Hz); IR (film, cm⁻¹) 3450, 2965, 2935, 2859, 1683, 1461, 1367, 1121, 1075.

### Example 6 Synthesis of S-tert Butyl 2,2-Difluoro-3-hydroxy-4,4-dimethylpentanethioate (XII)

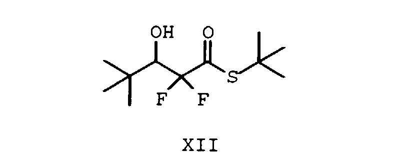

Following the procedure of Example 2, reaction of pivalaldehyde (0.119 mL, 1.10 mmol) affords compound (XII) (107 mg after isolation and purification, 42%) as a colorless oil, which was chromatographed using 10% EtOAc in hexane as eluent: ¹H NMR (CDCl₃) δ 1.01 (s, 9H), 1.47 (s, 9H), 2.42 (d, 1H), 3.77 (dt, 1H); ¹³C NMR (CDCl₃) δ 26.48, 29.36, 34.64, 48.78, 76.76 (t, J = 22 Hz), 117.38 (t, J = 262 Hz), 194.33 (t, J = 30 Hz); ¹⁹F NMR (CDCl₃) δ -104.84 (J_{FF} = 264 Hz, J_{FH} = 7 Hz), -118.04 (J_{FF} = 264, J_{FH} = 20 Hz); IR (film, cm⁻¹) 3520, 2960, 1677, 1597, 1460, 1374, 1164, 1064, 854.

### Example 7 Synthesis of S-tert Butyl 2,2-Difluoro-3-hydroxy-4-methylpentanethioate (XIII)

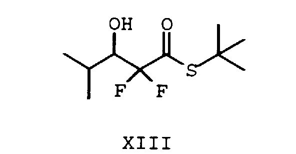

Following the procedure of Example 2, reaction of isobutyraldehyde (0.100 mL, 1.10 mmol) affords compound (XIII) (105 mg after isolation and purification, 44%) as a colorless oil, which was chromatographed using 10% EtOAc in hexane as eluent: ¹H NMR (CDCl₃) δ 0.99 (1, 6H), 1.49 (s, 9H), 1.95 (m, 1H), 2.42 (br, 1H), 3.81 (ddd, 1H); ¹³C NMR (CDCl₃) δ 16.84, 19.93, 28.44, 29.38, 49.96, 75.01 (t, J = 23 Hz), 116.44 (t, J = 261 Hz), 193.90 (t, J = 30 Hz); ¹⁹F NMR (CDCl₃) δ -110.50 (J_{FF} = 262 Hz, J_{FH} = 9 Hz), -117.80 (J_{FF} = 262, J_{FH} = 17 Hz); IR (film, cm⁻¹) 3474, 2967, 1677, 1479, 1466, 1367, 1163, 1064, 893.

### Example 8 Synthesis of S-tert Butyl 2,2-Difluoro-3-hydroxy-3-phenylbutanethioate (XIV)

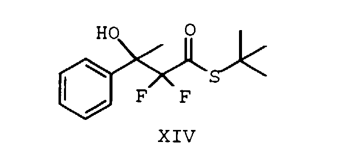

Following the procedure of Example 2, reaction of acetophenone (0.128 mL, 1.10 mmol) affords compound (XIV) (200 mg after isolation and purification, 69%) as a colorless oil, which was chromatographed using 10% EtOAc in hexane as eluent: ¹H NMR (CDCl₃) δ 1.35 (s, 9H), 1.72 (t, 3H), 3.39 (br, 1H), 7.33 (m, 3H), 7.50 (m, 2H); ¹³C NMR (CDCl₃) δ 23.24 (t, J = 3 Hz), 29.10, 49.00, 76.11 (t, J = 25 Hz), 114.80 (t, J = 265 Hz), 126.33, 128.04, 128.09, 139.49, 194.66 (t, J = 32 Hz); ¹⁹F NMR (CDCl₃) δ -114.26, -114.91 (J_{FF} = 259); IR (film, cm⁻¹) 3506, 2966, 1676, 1446, 1367, 1120, 1053, 884.

### Example 9 Synthesis of Bis(S-tert Butyl) 2,2,4,4-Tetrafluoro-3-hydroxy-3-phenylpentane-1,5-dithioate (XV)

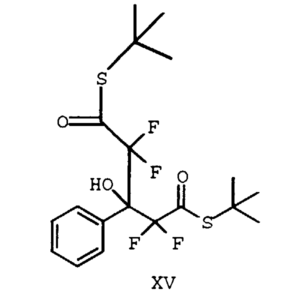

Following the procedure of Example 2, reaction of benzoyl chloride (0.128 mL, 1.10 mmol) affords compound (XV) (170 mg after isolation and purification, 77%) as a white solid, which was chromatographed using 10% EtOAc in hexane as eluent: mp 80 °C; ¹H NMR (CDCl₃) δ 1.35 (s,18H), 4.84 (br, 1H), 7.35 (m, 3H), 7.67 (m, 2H); ¹³C NMR (CDCl₃) δ 29.14, 49.43, 78.75 (t, J = 23 Hz), 113.44 (t, J = 271 Hz), 127.84, 127.93, 129.42, 131.35, 192.48 (t, J = 31 Hz); ¹⁹F NMR (CDCl₃) δ -109.93, -110.16 (J_{FF} = 261); IR (film, cm⁻¹) 3473, 2960, 1697, 1657, 1466, 1374, 1143, 966.

### Example 10 Synthesis of S-tert Butyl 1-(2-(2,2-difluoroethane-thioate))cyclohex-2-en-l-ol (XVI)

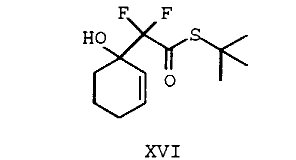

Following the procedure of Example 2, reaction of cyclohex-2-en-1-one (0.106 mL, 1.10 mmol) affords compound (XVI) (173 mg after isolation and purification, 65%) as a colorless oil, which was chromatographed using 15-20% EtOAc in hexane as eluent: ¹H NMR (CDCl₃) δ 1.50 (s, 9H), 1.72 (m, 1H), 1.80 (m, 1H), 2.03 (m, 1H), 2.31 (br, 1H), 5.77 (d, 1H), 6.10 (ddd, 1H); ¹³C NMR (CDCl₃) δ 17.42, 24.82, 29.48, 29.53, 49.12, 71.64 (t, J = 26 Hz), 115.98 (t, J = 263 Hz), 123.67, 135.39, 193.81 (t, J = 30 Hz); ¹⁹F NMR (CDCl₃) δ -115.76, -116.72 (J_{FF} = 257); IR (film, cm⁻¹) 3447, 2960, 2874, 1683, 1460, 1368, 1085.

### Example 11 Synthesis of D-erythro- and D-threo-2-Deoxy-2,2-difluoro-4,5-O-(1-ethylpropylidene)pentonic acid, tert-Butyl Thiolester (XVII, XVIII)

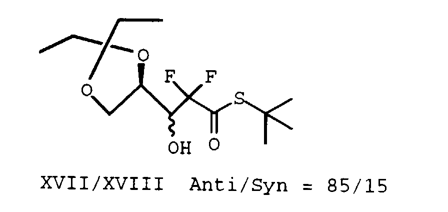

2,3-*O*-(3-pentylidene)-D-glyceraldehyde was synthesized by the procedure published in "2,3-*O*-(3-Pentylidene) -D-glyceraldehyde and 2,3-*O*-(3-Pentylidene)-L-glyceraldehyde: Convenient Glyceraldehyde Surrogates Obtained via a Novel Periodate-Based Oxidation System", Schmid, C.R.: Bradley, D.A Synthesis, 1992, 587-590.

Following the procedure of Example 2, reaction of 2,3-O-(3-pentylidene)-D-glyceraldehyde (0.155 mL, 1.00 mmol) affords a mixture of compounds XVII and XVIII. Chromatography of the residue with 5% and then 20% EtOAc/hexane as eluents gave *erytho-*XVII (172 mg, 53%) and *threo*-XVIII (37 mg, 11%) as colorless oils.

Data for e*rythro*-XVII: This compound was crystallized from hexane, mp 39-40 °C; ¹H NMR (CDCl₃) δ 0.87 (s, 6H), 1.50 (s, 9H), 1.60 (q, 2H), 1.62 (q, 2H), 2.47 (d, 1H), 3.96 (m, lH), 4.06 (m, lH), 4.23 (m, 1H), 4.32 (m, 1H); ¹³C NMR (CDCl₃) δ 7.90, 8.01, 28.94, 29.28, 29.40, 49.07, 65.45 (t, J = 3.8 Hz), 71.05 (t, J = 23 Hz), 73.56, 113.19, 115.21 (t, J = 260), 192.35 (t, J = 31 Hz); ¹⁹F NMR (CDCl₃) δ -115.3 (J_{FF} = 263 Hz, J_{FH} = 10 Hz), -117.5 (J_{FF} = 263 Hz, J_{FH} = 15 Hz); IR (film, cm⁻¹) 3427, 2973, 1683, 1460, 1209, 1177, 1137, 1078.

Data for *threo*-XVIII: ¹H NMR (CDCl₃) δ 0.88 (t, 3H), 0.89 (t, 3H), 1.50 (s, 9H), 1.63 (q, 2H), 1.66 (q, 2H), 2.90 (d, 1H), 3.81 (m, 1H), 3.94 (m, 1H), 4.10 (m, 1H), 4.31 (m, 1H) ; ¹³C NMR (CDCl₃) δ 7.97, 8.04, 29.02, 29.40, 29.52, 49.15, 66.53, 70.55 (J = 25 Hz), 72.36, 114.02, 114.7 (J = 263 Hz), 192.58 (J = 29 Hz); ¹⁹F NMR (CDCl₃) δ -108.78 (J_{FF} = 267 Hz, J_{FH} = 6 Hz), -120.01 (J_{FF} = 267, J_{FH} = 16 Hz); IR (film, cm⁻¹) 3533, 3453, 2973, 1677, 1453, 1170, 1137, 1084.

## Claims

1. α,α-Difluoro-β-hydroxy thiol esters of formula (III) 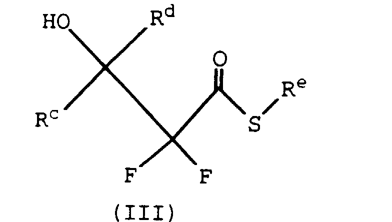 wherein
R^{c} and R^{d} are independently selected from the group consisting of H, C₁-C₆ alkyl, substituted C₁-C₆ alkyl, *S-tert*-butyl difluorothioacet-2-yl, C₃-C₈ cycloalkyl, C₃-C₈ cycloalkenyl, substituted C₃-C₈ cycloalkyl, substituted C₃-C₈ cycloalkenyl, aryl, substituted aryl, C₆-C₁₀ fused aromatic rings, and substituted C₆-C₁₀ fused aromatic rings; or
R^{c} and R^{d} together make up a ring selected from the group consisting of C₃-C₈ cycloalkyl, C₃-C₈ cycloalkenyl, substituted C₃-C₈ cycloalkyl and substituted C₃-C₈ cycloalkenyl;
R^{e} is selected from the group consisting of C₁-C₁₀ alkyl, substituted C₁-C₁₀ alkyl, aryl, substituted aryl, C₃-C₈ cycloalkyl, C₃-C₈ cycloalkenyl, substituted C₃-C₈ cycloalkyl and substituted C₃-C₈ cycloalkenyl, C₆-C₁₀ fused aromatic rings, and substituted C₆-C₁₀ fused aromatic rings.

2. An α,α-difluoro-β-hydroxy thiol ester of Claim 1 in which R^{e} is tert-butyl.

3. An α,α-difluoro-β-hydroxy thiol ester of Claim 1 in which R^{c} is 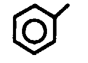 and R^{d} is H, methyl, or *S-tert*-butyl difluorothioacet-2-yl.

4. A difluoroethanethioate compound of formula (IV); 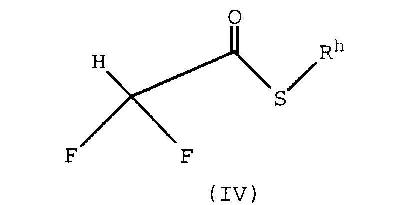 wherein
R^{h} is selected from the group consisting of C₃-C₈ cycloalkenyl and substituted C₃-C₈ cycloalkenyl.

5. A process to make α,α-difluoro-β-hydroxy thiol esters of formula (IIIA), 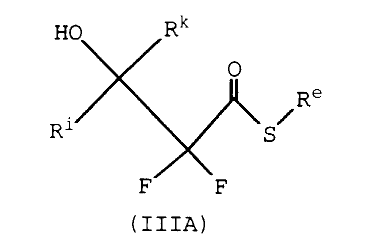 wherein
Rⁱ and R^{k} are independently selected from the group consisting of H, C₁-C₆ alkyl, substituted C₁-C₆ alkyl, *S-tert*-butyl difluorothio-acet-2-yl, C₃-C₈ cycloalkyl, C₃-C₈ cycloalkenyl, substituted C₃-C₈ cycloalkyl, substituted C₃-C₈ cycloalkenyl, aryl, substituted aryl, 1,3-dioxolan-4-yl, substituted 1,3-dioxolan-4-yl, C₆-C₁₀ fused aromatic rings, substituted C₆-C₁₀ fused aromatic rings; or
Rⁱ and R^{k} together make up a ring selected from the group consisting of C₃-C₈ cycloalkyl, C₃-C₈ cycloalkenyl, substituted C₃-C₈ cycloalkyl, and substituted C₃-C₈ cycloalkenyl;
R^{e} is selected from the group consisting of C₁-C₁₀ alkyl, substituted C₁-C₁₀ alkyl, aryl, substituted aryl, C₃-C₈ cycloalkyl, C₃-C₈ cycloalkenyl, substituted C₃-C₈ cycloalkyl, substituted C₃-C₈ cycloalkenyl, C₆-C₁₀ fused aromatic rings, and substituted C6-C10 fused aromatic rings; comprising reacting a difluoroethanethioate of formula (IVA) 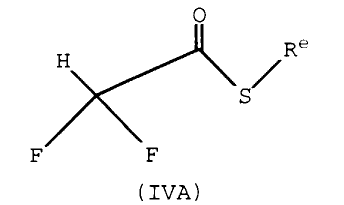 wherein
R^{e} is as defined previously;
with a second reactant selected from the group consisting of aldehydes, ketones, acid halides and esters; in a solvent and in the presence of a strong base; with the proviso that the process is conducted in the absence of a catalyst and in the absence of a silyl containing compound.

6. The process of Claim 5 in which said second reactant is an aldehyde or ketone of formula (V) 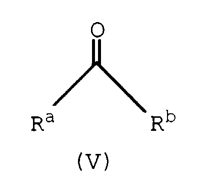 wherein R^{a} and R^{b} are independently selected from the group consisting of H, C₁-C₆ alkyl, substituted C₁-C₆ alkyl, C₃-C₈ cycloalkyl, C₃-C₈ cycloalkenyl, substituted C₃-C₈ cycloalkyl, substituted C₃-C₈ cycloalkenyl, aryl, substituted aryl, 1,3-dioxolan-4-yl, substituted 1,3-dioxolan-4-yl, C₆-C₁₀ fused aromatic rings and substituted C₆-C₁₀ fused aromatic rings; or R^{a} and R^{b} together make up a ring selected from the group consisting of C₃-C₈ cycloalkyl, C₃-C₈ cycloalkenyl, substituted C₃-C₈ cycloalkyl and substituted C₃-C₈ cycloalkenyl.

7. The process of Claim 5 in which said second reactant is an acid halide of formula (VI) 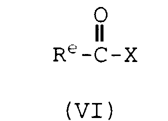 where X is chloro, bromo, fluoro or iodo and
R^{e} is selected from the group consisting of C₁-C₁₀ alkyl, substituted C₁-C₁₀ alkyl, aryl, substituted aryl, C₃-C₈ cycloalkyl, C₃-C₈ cycloalkenyl, substituted C₃-C₈ cycloalkyl, substituted C₃-C₈ cycloalkenyl, C₆-C₁₀ fused aromatic rings, and substituted C₆-C₁₀ fused aromatic rings.

8. The process of Claim 5 in which said second reactant is an ester of formula (VII) 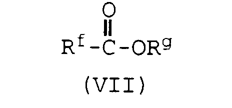 where R^{f} and R^{g} are independently R^{e}, where R^{e} is selected from the group consisting of C₁-C₁₀ alkyl, substituted C₁-C₁₀ alkyl, aryl, substituted aryl, C₃-C₈ cycloalkyl, C₃-C₈ cycloalkenyl, substituted C₃-C₈ cycloalkyl, substituted C₃-C₈ cycloalkenyl, C₆-C₁₀ fused aromatic rings, and substituted C₆-C₁₀ fused aromatic rings.

9. The process of Claim 5 in which said α,α-difluoroethanethioate is S-tert-butyl difluoroethanethioate and said second reactant is 2,3,-O-(3-pentylidene)-D-glyceraldehyde.

10. A process to make gemcitabine hydrochloride, the improvement characterized in that the lactone intermediate, 2-deoxy-2,2-difluoro-D-erythro-pentofuranose-1-ulose-3,5-dibenzoate: 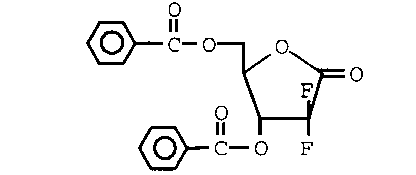 is made from D-erythro-2-Deoxy-2,2-difluoro-4,5-O-(1-ethylpropylidene) pentoic acid, tert-Butyl thioester, with said D-erythro-2-Deoxy-2,2-difluoro-4,5-O-(1-ethylpropylidene) pentoic acid, tert-Butyl thioester being made by the process of Claim 5.
